# EUROPEAN PATENT APPLICATION

(11) **EP 4 768 173 A1**
(43) Date of publication of application: **01.07.2026**
(21) Application number: 24858182.9
(22) Date of filing: 29.07.2024
(51) Int. Cl.: B23P 21/00, A61M 5/178, A61M 5/32

(54) **ASSEMBLY METHOD FOR PREFILLED SYRINGE**

(30) Priority: 25.08.2023 CN 202311079664
(71) Applicant: Shanghai Innopac Medical Technology Co., Ltd., Shanghai 201605 (CN)
(72) Inventor: GUO, Rong, Shanghai 201605 (CN)
(74) Representative: IPrime Bonnekamp Sparing Patentanwaltsgesellschaft mbH
(86) International application number: PCT/CN2024/108076
(87) International publication number: WO 2025/044639

(57) **Abstract**

The present invention belongs to the technical field of medical devices, and particularly relates to an assembly method for assembling a prefilled syringe. The assembly method of the present invention comprises the following steps: S01. sleeve the needle sleeve onto the needle seat, and use a camera to confirm whether the needle sleeve is punctured by the needle; S02. insert a sleeve pressure head into the protective cover to radially push out the stop hook, wherein the minimum inner diameter of the sleeve press head is greater than the maximum outer diameter of the needle sleeve; S03. sleeve the protective cover with the sleeve press head inserted therein onto the needle seat; S04. remove the sleeve pressure head from the protective cover. This method uses the sleeve to expand the protective cover from inside to outside, avoiding the risk that the protective cover may get stuck on the needle sleeve or the needle seat during later installation, thereby achieving the purpose of first installing the needle sleeve and using a camera to check whether it is punctured, and then installing the protective cover.

## Description

The invention belongs to the technical field of medical devices, and in particular relates to an assembling method of a prefilled syringe.

### Background of the invention

Prefilled syringes are injection devices used in the medical field that integrate the functions of a syringe and a medication container. These syringes are prefilled with medication during the manufacturing process and sealed to protect the drug's purity and effectiveness. This prefilled design makes use more convenient and quicker for medical staff, reducing the time and steps required for medication preparation. Furthermore, because prefilled syringes are singleuse, they avoid the risk of crossinfection and enhance patient safety. Furthermore, strict sealing measures are implemented during the manufacturing process to effectively protect the drug's purity and stability, extending its shelf life.

The main components of a prefilled syringe include the syringe body, medication container, piston, needle, and protective cap. The syringe body is typically made of transparent material, allowing medical personnel to clearly observe the dosage and status of the medication solution. The medication container is typically made of glass or plastic, with a capacity tailored to the needs, allowing for different dosages of medication. The piston pushes the medication into the needle for injection. The needle then delivers the medication into the patient. The protective cap protects the needle before and after use.

However, existing prefilled syringes are often discarded after use, leaving the needle exposed and dangerous. To address this issue, operators often replace the removed protective cap. However, this approach has many drawbacks. First, the protective cap is custom made for the needle, with a small opening, making it difficult to reinsert after removal. Second, the reinstallation process can easily result in the needle pricking the operator's hand.

To solve the above problems, a protective prefilled syringe with a protective cover has been proposed on the market. The inventor has previously applied for an invention patent in the related field, specifically CN114642793A, a prefilled syringe with a front protective cover. The protective cover can adjust the axial position by pushing and pulling, which is used to expose the needle to complete the injection, and to cover the needle and discard it after the injection is completed to avoid needle sticks to others.

Regarding the assembly method of the prefilled syringe assembly, CN114652920A records a method for assembling a prefilled syringe assembly with a protective cover, which specifically includes the following steps:
S1. Insert the needle sleeve unit axially into the protective cover unit;
S2. The protective cover unit with the needle sleeve unit is axially sleeved on the mounting portion;
S3. Push the piston into the syringe;
S4. Insert the push rod onto the piston.

However, the above assembly method has a drawback: during step S2, the needle may pierce the needle sleeve unit. This puncture will produce a large amount of rubber debris, which is unacceptable for medical applications because it compromises the sterile environment. Typically, on the production line, a camera is used to confirm whether the needle cover has been punctured, allowing prefilled syringes with punctured needle covers to be removed promptly. However, due to the obstruction of the protective cover unit, it is difficult for workers to visually confirm whether the needle sleeve unit is punctured, making it impossible to promptly remove prefilled syringes with punctured needle sleeves from the production line, thereby creating a certain degree of medical or commercial risk.

In summary, there is currently a need for a prefilled injection needle assembly method that can easily confirm whether the needle sleeve is pierced by the needle during the assembly process and can also smoothly install a protective cover on the outside of the needle sleeve.

### Description of the invention

The present invention provides a method for assembling a prefilled syringe. This method uses a sleeve to expand the protective cover from the inside out, avoiding the risk of the protective cover becoming stuck on the needle sleeve or needle seat during subsequent installation. This method allows the needle sleeve to be installed first, checked for punctures using a camera, and then the protective cover installed.

The technical solution adopted by the present invention to solve the above problem is: an assembling method of a prefilled syringe, wherein the prefilled syringe comprises:
A syringe barrel, used to contain liquids;
A needle seat, mounted at the distal end of the syringe barrel;
a needle, mounted on the needle seat and connected to the syringe barrel to allow the liquid to flow;
a protective cover, at least partially sleeved on the syringe barrel and at least partially sleeved on the needle seat;
a needle sleeve at least partially sleeved on the needle seat to completely sleeve the needle;
The proximal end of the protective cover is provided with a stop hook which contracts radially inwards and is used to push up the proximal end surface of the needle seat;
The assembly method includes the following steps:
   S01. The needle sleeve is sleeved on the needle seat, and a camera is used to confirm whether the needle sleeve is pierced by the needle;
   S02. The sleeve pressure head is inserted into the protective cover for radially outwardly pushing up the stop hook, wherein the minimum inner diameter of the sleeve pressure head is greater than the maximum outer diameter of the needle sleeve;
   S03. The protective cover, with the sleeve pressure head already inserted, is sleeved onto the needle seat;
   S04. The sleeve pressure head is removed from the protective cover.

A further preferred technical solution is that the minimum inner diameter of the sleeve pressure head is greater than the maximum outer diameter of the needle sleeve.

A further preferred technical solution is that the minimum outer diameter of the sleeve pressure head is greater than the maximum outer diameter of the needle seat.

A further preferred technical solution is that the wall thickness of the sleeve pressure head is 500-700 µm.

A further preferred technical solution is that the proximal end of the protective cover is further provided with a cantilever for pushing up the distal end surface of the needle seat.

A further preferred technical solution is that: a cantilever groove is provided on the stop hook, and the cantilever is arranged on the inner side surface of the distal end of the cantilever groove.

A further preferred technical solution is that: a contraction ring for engaging with the distal end surface of the needle seat is provided at the distal end of the protective cover.

A further preferred technical solution is that: a cantilever hook for engaging with the distal end surface of the needle seat is provided at the distal end of the protective cover.

A further preferred technical solution is that the sleeve pressure head material includes one or more combinations of polypropylene, polyethylene, and polystyrene.

A further preferred technical solution is that a stop protrusion is further provided at the distal end of the sleeve pressure head.

### Beneficial effects

In summary, the present invention has the following advantages:
1. By utilizing the sleeve pressure head, the effect of installing the needle sleeve first and then the protective cover can be achieved. Thus, after the needle sleeve is sleeved onto the needle seat, it can be timely checked whether the needle sleeve has been pierced by the needle, thereby improving the yield rate of prefilled syringes on the production line.
2. The wall thickness range of the sleeve is precisely defined, which not only meets the functions of supporting the cantilever and the stop hook, but also prevents touching the needle sleeve during the sleeving process, avoiding the risk of the needle sleeve being pierced by the needle during the secondary installation process.

### Brief description of the drawings

FIG. 1 is a schematic diagram of the needle sleeve being sleeved on the needle seat in step S01 .
FIG. 2 is a schematic diagram showing that the sleeve pressure head is aligned with the protective cover but not inserted in step S02 .
FIG. 3 is a schematic diagram of inserting the sleeve pressure head into the protective cover in step S02 .
FIG. 4 is a schematic diagram of sleeving the protective cover, into which the sleeve pressure head has been inserted, onto the needle seat in step S03.
FIG. 5 is a schematic diagram of removing the sleeve pressure head from the protective cover in step S04 .
FIG. 6 is a schematic structural diagram of the protective cover in the first embodiment.

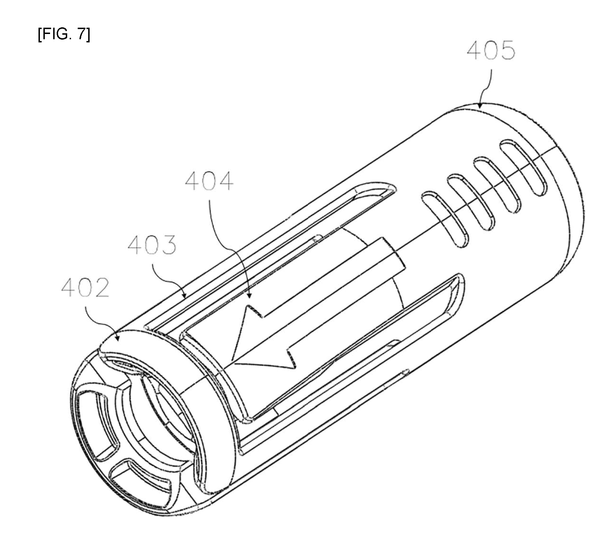

FIG. 7 is a schematic diagram of the protective cover in FIG. 6 rotated 180 degrees.

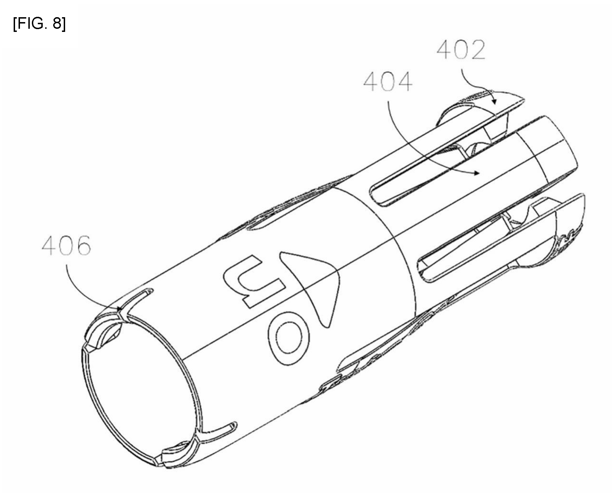

FIG. 8 is a schematic structural diagram of the protective cover in the second embodiment.

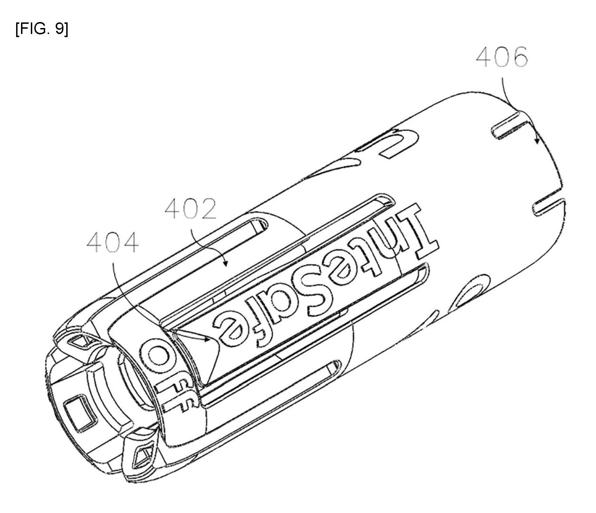

FIG. 9 is a schematic diagram of the protective cover in FIG. 8 rotated 180 degrees.

In the accompanying drawings, the components represented by the respective reference numerals are as follows: syringe barrel 1, needle seat 2, needle 3, protective cover 4, needle sleeve 5, stop hook 402, cantilever groove 403, cantilever 404, contraction ring 405, cantilever hook 406, stop protrusion 601.

### Description of a preferred embodiment

1. Distal end: refers to the syringe and the various components installed on the syringe in a normal manner, which faces toward the one end of the needle.
2. Proximal end: refers to the syringe and the various components installed on the syringe in a normal manner, which is away from the one end of the needle.
3. Axis: refers to the line connecting the centroids of all cross sections of a component with a symmetrical cross section.
4. Open: refers to the ability to expose the needle or needle sleeve during operation.
5. Closed: refers to the ability to cover the needle or needle sleeve during operation.
6. Sleeving: refers to the act of installing component one on the outer surface of component two when the inner diameter of component one is larger than the outer diameter of component two.
7. Setting: refers to any method used to combine two parts together, such as bonding, snapping, welding, screwing, placement, etc.
8. Pushing up: refers to component one exerting a force on component two, causing component two to move in a direction opposite to the position of component one.
9. Minimum/Maximum Diameter: For components with a distinct axis, a cross-section perpendicular to the axis is taken. If the cross-section is approximately circular or polygonal, the narrowest portion of the cross-section is the minimum diameter of the component, and the widest portion of the cross-section is the maximum diameter. The minimum diameter of a given cross-section should not be greater than its maximum diameter.

The present invention will be further described in detail below with reference to the accompanying drawings.

This specific embodiment is merely an explanation of the present invention and is not intended to limit the present invention. After reading this specification, those skilled in the art may make non-creative modifications to this embodiment as needed. However, as long as such modifications are within the scope of the claims of the present invention, they are protected by patent law.

### Embodiment 1

In this embodiment, a method for assembling a prefilled syringe is provided, wherein the prefilled syringe includes: a syringe barrel 1, a needle seat 2, a needle 3, a protective cover 4, and a needle sleeve 5, wherein the protective cover 4 is provided with a stop hook 402, a cantilever groove 403, a cantilever 404, and a contraction ring 405.

The minimum inner diameter of the sleeve pressure head 6 is greater than the maximum outer diameter of the needle sleeve 5 .

The minimum outer diameter of the sleeve pressure head 6 is greater than the maximum outer diameter of the needle seat 2 .

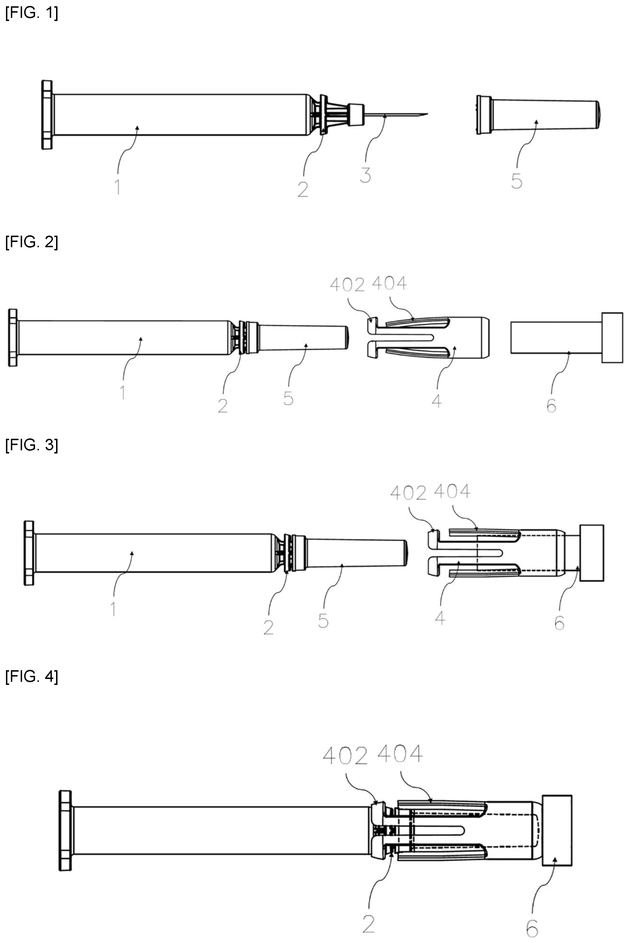

Referring to FIG. 1 , the needle sleevesleeve is shown being sleeved onto the needle seat in step S01 , wherein the basic structure of the prefilled syringe can also be seen, including:
Syringe barrel 1, used for containing liquid;
The needle seat 2 is mounted at the distal end of the syringe barrel 1;
a needle 3 mounted on the needle seat 2 and connected to the syringe barrel 1 to allow the liquid to flow;
The needle sleeve 5 is at least partially sleeved on the needle seat 2 to completely sleeve the needle 3 .

Referring to FIG. 1, in step S01, the needle sleeve 5 is sleeved onto the needle seat 2 to completely cover the needle 3. This process is completed using conventional production line equipment for prefilled syringes. First, on the production line, the syringe and needle sleeve undergo pretreatment steps such as cleaning and disinfection to ensure product hygiene and quality. The syringe is then positioned in a fixed position, typically controlled by a robotic arm or conveyor system. The needle sleeve is then prepared and automatically supplied to the position of the sleeve assembly, typically via a feeding system or vibrating plate. Next, the sleeving process begins. Once the syringe and needle sleeve are ready, the sleeving device precisely inserts the needle sleeve into the needle tip of the syringe. This process may involve mechanical operations, such as rotation and pressure, to ensure a secure connection between the needle sleeve and the syringe.

Specifically, after the sleeving is completed, the syringe undergoes a quality inspection to ensure that the needle sleeve 5 has not been punctured by the needle 3. This inspection process utilizes conventional cameras. In this embodiment, one or more high-resolution industrial cameras are installed on the assembly line, typically connected to image processing software and a control system. The syringe is positioned within the camera's field of view, typically controlled by a conveyor system or robotic arm. The camera captures images of the syringe's needle tip. These images can include different angles and lighting conditions, allowing for a more comprehensive inspection of the sleeving connection. The captured images are then transmitted to image processing software for analysis. The software uses algorithms and pattern recognition techniques to detect whether the needle sleeve is pierced by the needle. For example, methods such as edge detection, shape matching, or color analysis can be used to identify anomalies. Based on the image processing results, the system determines whether each syringe's sleeve has passed quality inspection. If an anomaly is detected, an alarm may be triggered or the unqualified product may be removed from the assembly line. Furthermore, the system typically records the inspection results for each syringe and generates statistical data for quality control and improvement.

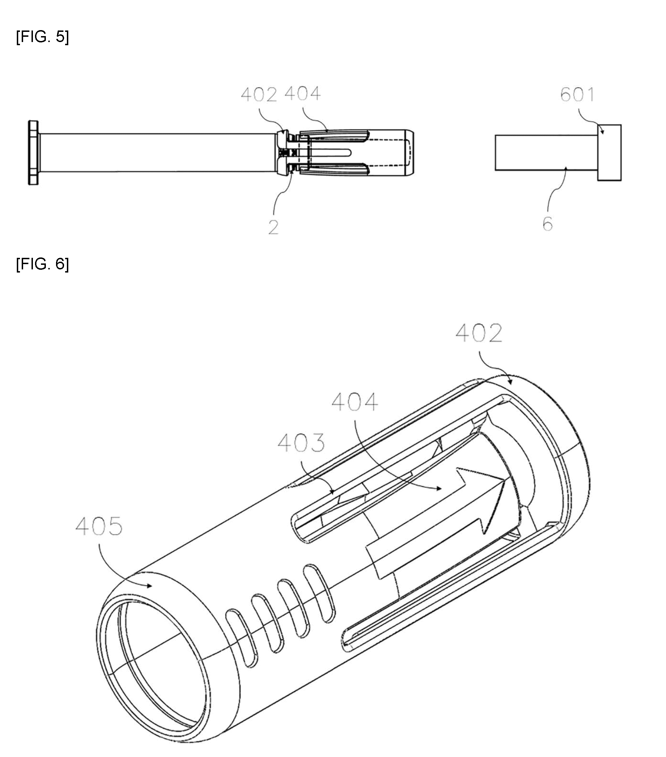

Referring to FIG. 2, a schematic diagram is shown in which the sleeve pressure head is aligned with the protective cover but not inserted in step S02. It can be seen that the protective cover 4 is at least partially sleeved on the syringe barrel 1 and at least partially sleeved on the needle seat 2; a sleeve pressure head 6 is also provided coaxially with the protective cover 4, and the sleeve pressure head 6 can be inserted into the protective cover 4. As can be seen in the figure, the needle seat 2 has a radially outwardly expanding ridge, which will hinder the installation process of the protective cover 4. The specific structure of the protective cover 4 is shown in FIG. 6. It can be seen that the proximal end of the protective cover 4 is provided with a radially inwardly contracted stop hook 402 for pushing up the proximal surface of the needle seat 2. The proximal end of the protective cover 4 is also provided with a cantilever 404 for pushing up the distal end surface of the needle seat 2. The way in which the protective cover 4 realizes its function can be referred to CN114642793A.

The radially outwardly flared ridges of the needle seat 2 can block the stop hooks 402 or cantilevers 404 of the protective cover 4 during installation, making the installation process of the protective cover 4 not smooth enough. Furthermore, the inwardly contracted stop hooks 402 or cantilevers 404 can contact the needle sleeve 5 during sleeving installation process, creating a risk that the needle 3 could pierce the needle sleeve 5. This would render the camera inspection of the needle sleeve 5 in step S01 meaningless. Therefore, in this embodiment, before sleeving the protective cover 4 onto the needle seat 2, referring to FIG. 3 , the sleeve pressure head 6 is first inserted into the protective cover 4 from the distal end, with the stop hooks 402 and cantilevers 404 facing the distal end. This allows the sleeve pressure head 6 to follow the inclined surfaces of the stop hooks 402 and cantilevers 404 to squeeze them radially outward during insertion. Furthermore, the minimum inner diameter of the sleeve pressure head 6 is greater than the maximum outer diameter of the needle sleeve 5. Therefore, even when the sleeve pressure head 6 and the protective cover 4 together sleeve the needle sleeve 5, the sleeve pressure head 6 theoretically does not come into contact with the needle sleeve 5. That is, the needle sleeve 5 that was not pierced in step S01 remains intact during the above sleeving process. Furthermore, the minimum outer diameter of the sleeve pressure head 6 is greater than the maximum outer diameter of the needle seat 2. The maximum outer diameter of the needle seat 2 is the maximum outer diameter of the radially outwardly extending ridge of the needle seat 2. When the minimum outer diameter of the sleeve pressure head 6 is greater than the maximum outer diameter of the needle seat 2, the stop hook 402 and cantilever 404 radially outwardly pressed by the sleeve pressure head 6 can easily pass over the ridge, i.e., they will not be blocked by the needle seat 2. Referring to FIG. 4 , when the protective cover 4 is fully sleeved on the needle sleeve 5, the stop hook 402 can completely pass over the ridge of the needle seat 2.

In addition, referring to FIG. 4 , when the sleeve pressure head 6 is not long enough to directly contact the stop hook 402, although it can be seen in the figure that the sleeve pressure head 6 does not directly push up the stop hook 402 radially outward, but only pushes up the cantilever 404 radially outward, since the cantilever 404 is installed in the cantilever groove 403 provided on the stop hook 402, when the sleeve pressure head 6 pushes the cantilever 404 radially outward, it also acts on the stop hook 402 to a certain extent. When the sleeve pressure head 6 is long enough to directly contact the stop hook 402, the sleeve pressure head 6 can contact the stop hook 402 and the cantilever 404 at the same time, and the stop hook 402 does not need to be driven by the cantilever 404.

Referring to FIG. 5, a schematic diagram illustrating the removal of the sleeve pressure head from the protective cover in step S04 is shown. Once the protective cover 4 is completely sleeved in the needle sleeve 5, the sleeve pressure head 6 can be immediately withdrawn distally. At this point, the stop hook 402 and cantilever 404 return to their original radially inwardly contracted positions, respectively firmly engaging the proximal and distal sides of the ridge of the needle seat 2.

In addition, referring to FIG. 4, a stop protrusion 601 is also provided on the distal side of the sleeve pressure head 6. The function of the stop protrusion 601 is to facilitate the movement of the sleeve pressure head 6 and prevent the sleeve pressure head 6 from being fully inserted into the protective cover 4 during the insertion process and unable to be pulled out in step S04.

### Embodiment 2

In this embodiment, the assembly method of the prefilled syringe is basically the same as that of the first embodiment, except that:
The protective cover 4 is provided with a cantilever hook 406.

The wall thickness of the sleeve pressure head 6 is 500-700 µm.

The material of the sleeve pressure head 6 includes one or more combinations of polypropylene, polyethylene, and polystyrene.

In this embodiment, the cantilever hook 406 engages the ridge of the needle seat 2, preventing the protective cover 4 from fully reaching the syringe barrel 1. Its function is similar to that of the contraction ring 405, but the cantilever hook 406 also tends to retract inward, blocking the sleeve pressure head 6 when it is inserted into the protective protective cover 4. In this embodiment, the sleeve pressure head 6 has a wall thickness of 500-700 µm, which provides sufficient strength while not being too thick to contact the needle sleeve 5 when inserted into the protective cover 4. Alternatively, the sleeve pressure head 6 could be made of a metal material, such as stainless steel, to achieve a similar effect.

### Embodiment 3

In this embodiment, the assembly method of the prefilled syringe described in Embodiment 1 is used, the sleeve pressure head 6 is made of stainless steel, and sleeve pressure heads 6 with different wall thicknesses are used for testing. The test results are shown in the following table:

| Wall Thickness (µm) | Whether the Stop Hook is Pushed Up | Whether the Cantilever Arm is Pushed Up | Whether it Contacts the Needle Sleeve |
|---|---|---|---|
| 300 | No | No | No |
| 400 | No | Yes | No |
| 500 | Yes | Yes | No |
| 600 | Yes | Yes | No |
| 700 | Yes | Yes | No |
| 800 | Yes | Yes | Yes |

Among them, it can be seen that when the wall thickness of the sleeve pressure head 6 is 300 µm, although it will not contact the needle sleeve 5, its strength is not enough to push up the stop hook 402 and the cantilever 404, and it will deform under the radial contraction force of the stop hook 402 and the cantilever 404; when the wall thickness of the sleeve pressure head 6 is 400 µm, although the strength of the sleeve pressure head 6 can push up the cantilever 404, the strength of its proximal end is not enough to lift the stop hook 402, and when the wall thickness of the sleeve pressure head 6 is between 500 and 700 µm, the strength of the sleeve pressure head 6 can push up the stop hook 402 and the cantilever 404 at the same time, and its inner side will not contact the needle sleeve 5; when the wall thickness of the sleeve pressure head 6 is 800 µm, although the strength of the sleeve pressure head 6 can lift the stop hook 402 and the cantilever 404 at the same time, due to its excessive wall thickness, it will contact the needle sleeve 5 during the insertion process, and there is a risk of the needle 3 piercing the needle sleeve 5.

In addition, the same or similar reference numerals are used in the drawings and description to refer to the same or similar parts or steps whenever possible. The drawings are presented in simplified form and are not drawn to exact scale. For convenience and clarity only, directional terms such as top, bottom, left, right, upward, above, over, below, downward, rear, and front may be used in the drawings. These and similar directional terms should not be construed as limiting the scope of this disclosure in any way.

## Claims

1. A method for assembling a prefilled syringe, wherein the prefilled syringe comprises: a syringe barrel (1), used for containing liquid; a needle seat (2) mounted at a distal end of the syringe barrel (1);a needle (3) mounted on the needle seat (2) and connected to the syringe barrel (1) to allow the liquid to flow; a protective cover (4) at least partially sleeved on the syringe barrel (1) and at least partially sleeved on the needle seat (2);a needle sleeve (5) at least partially sleeved on the needle seat (2) to completely sleeve the needle (3);wherein a proximal end of the protective cover (4) is provided with a stop hook (402) which contracts radially inwards and is used to push up a proximal end surface of the needle seat (2);**Characterized in that** the assembly method comprises the following steps: S01. Sleeving the needle sleeve (5) on the needle seat (2);S02. Inserting a sleeve pressure head (6) into the protective cover (4) to radially outwardly push up the stop hook (402);S03. sleeving the protective cover (4), into which the sleeve pressure head (6) has been inserted,on the needle seat (2);S04. removing the sleeve pressure head (6) from the protective cover (4).

2. The method for assembling a prefilled syringe according to claim 1, **characterized in that**: a minimum inner diameter of the sleeve pressure head (6) is greater than the maximum outer diameter of the needle sleeve (5).

3. The method for assembling a prefilled syringe according to claim 1, **characterized in that**: a minimum outer diameter of the sleeve pressure head (6) is greater than the maximum outer diameter of the needle seat (2).

4. The method for assembling a prefilled syringe according to claim 1, **characterized in that**: a wall thickness of the sleeve pressure head (6) is 500-700 µm.

5. The method for assembling a prefilled syringe according to claim 1, **characterized in that**: the proximal end of the protective cover (4) is further provided with a cantilever (404) for pushing up a distal end surface of the needle seat (2).

6. The method for assembling a prefilled syringe according to claim 5, **characterized in that**: the stop hook (402) is provided with a cantilever groove (403), and the cantilever (404) is arranged on an inner side surface of a distal end of the cantilever groove (403).

7. The method for assembling a prefilled syringe according to claim 1, **characterized in that**: a contraction ring (405) for engaging with the distal end surface of the needle seat (2) is provided at a distal end of the protective cover (4).

8. The method for assembling a prefilled syringe according to claim 1, **characterized in that**: A cantilever hook (406) is provided at a distal end of the protective cover (4) for engaging with the distal end surface of the needle seat (2).

9. The method for assembling a prefilled syringe according to claim 1, **characterized in that**: the material of the sleeve pressure head (6) includes one or more of polypropylene, polyethylene, and polystyrene.

10. The method for assembling a prefilled syringe according to claim 1, **characterized in that**: a stop protrusion (601) is further provided at a distal end of the sleeve pressure head (6).
